# FASCICULE DE BREVET EUROPEEN

(11) **EP 0 375 471 B1**
(45) Date de publication et mention de la délivrance du brevet: **10.04.1996**
(21) Numéro de dépôt: 89402687.1
(22) Date de dépôt: 29.09.1989
(51) Int. Cl.: A61K 31/645, C07D 219/10

(54) **Utilisation de 1,2,3,4-tétrahydro-acridines pour le traitement du SIDA, et composés**
Verwendung von 1,2,3,4-Tetrahydroacridinen zur Behandlung von AIDS, sowie Verbindungen
Use of 1,2,3,4-tetrahydro-acridines in the treatment of AIDS, and compounds

(30) Priorité: 19.12.1988 FR 8816749
(43) Date de publication de la demande: 27.06.1990
(73) Titulaire: STE CIVILE DE RECHERCHE NEWPHARM, F-78000 Versailles (FR)
(72) Inventeur: Dietlin, François, F-78110 Le Vésinet (FR); Fredj, Danièle, F-91190 Gif s/Yvette (FR)
(74) Mandataire: Burtin, Jean-François

(56) Documents cités:
- EP-A- 0 306 825
- WO-A-88/03804
- WO-A-88/05657
- WO-A-88/07854
- WO-A-90/14086
- JOURNAL DE CHIMIE PHYSIQUE, vol. 78, no. 6, 1981, pages 527-530; R. FAURE et al.: "Etude par résonance magnétique nucléaire du carbone 13 de quelques tetrahydro-1,2,3,4 acridines substituées en position 9"
- THE LANCET, vol. 1, 16 avril 1988, page 887; D.J. AMES et al.: "Hepatotoxicity of tetrahydroacridine"
- INDIAN JOURNAL OF CHEMISTRY, vol. 26b, no. 4, avril 1987, pages 330-334, Publications & Information Directorate, CSIR, New Delhi, IN; P. ASTHANA et al.: "Synthesis of 9-Substituted-oxy Acridine & 1,2,3,4-tetrahydroacridine derivatives as potential biodynamic agents"
- INTERNATIONAL JOURNAL OF CLINICAL PHARMACOLOGY THERAPY AND TOXICOLOGY, vol. 27, no. 8, août 1989, pages 408-410; G. FREDJ et al.: "Tetrahydroaminoacridine in HIV infections"
- BIOMEDICINE & PHARMACOTHERAPY, vol. 43, no. 4, 1989, pages 235-236, Elsevier, Paris, FR; G. MATHé et al.: "Amino acridines action on Friend's retrovirus in relation to their molecular ionization"
- 6TH INTERNATIONAL CONFERENCE AIDS, 20th-23rd June 1990, abrégé de conférence no. S.B. 458, pages 200-201; G. FREDJ et al.: "Follow-up of Hiv infected post Azt patients treated by tacrine (THA)
- 6TH INTERNATIONAL CONFERENCE AIDS, 20th-23rd June 1990, abrégé de conférence no. S.B. 457, pages 200-201; M. YOULE et al.: "Open study of tacrine hydrochloride in Hiv P24 antigen positive patients"

## Description

La présente invention se rapporte au domaine de la chimie thérapeutique et plus particulièrement à celui de la thérapeutique immunologique.

Elle a plus particulièrement pour objet l'utilisation de produits ou de compositions qui permettent d'assurer seuls ou en association avec un autre principe actif la manufacture d'un médicament destiné au traitement des syndromes immuno-déficitaire acquis ou non.

Elle a spécifiquement pour objet l'utilisation de compositions pharmaceutiques pour la manufacture d'un médicament assurant la régénération des lymphocytes T4 ou l'augmentation du nombre des lymphocytes T4 chez des sujets présentant un syndrome immuno-déficitaire, caractérisées en ce qu'elles renferment, à titre de principe actif, la 9-amino 1,2,3,4-Tetrahydroacridine de formule : 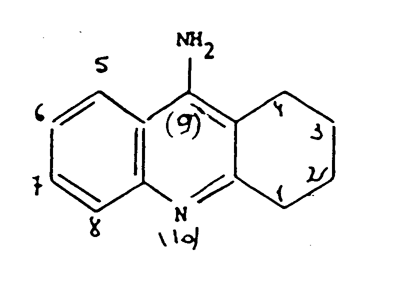
sous forme libre ou salifiée
ou un de ses précurseurs biologiques
en association ou en mélange avec un véhicule ou un excipient inerte non-toxique, pharmaceutiquement-acceptable.

Dans ce qui suit, les sels de 9-amino tétrahydroacridine ou de ses dérivés sont ceux obtenus par addition avec un acide minéral ou organique thérapeutiquement compatible, comme les chlorhydrate, bromhydrate, sulfate, nitrate, phosphate, sulfite, thiosulfate, acétate, butyrate, caproate, subérate, succinate, tartrate, citrate, glutamate, aspartate, benzoate, gentisate, salicylate, triméthoxybenzoate, vanillinate, eugenate, nicotinate, naphtoate, benzenesulfonate, méthanesulfonate, isethionate, ethanesulfonate, p.toluène sulfonate, camphosulfonate, naphtalène sulfonate, glucose 1-phosphate et le glucose 1,6-diphosphate.

Les précurseurs biologiques de la 9-amino 1,2,3,4-Tétrahydro acridine sont notamment les mono- et diacylamines comme par exemple :
- les composés de formule 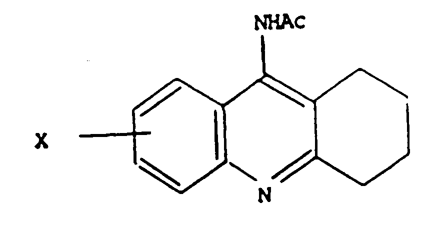 dans laquelle Ac est un reste acyle dérivé d'un acide organique carboxylique ayant de 1 à 10 atomes de carbone comme l'acétyle, propionyle ou benzoyle, 2,6-dichlorobenzoyle, 3,4,5-trimethoxy benzoyle, cinnamoyle, nicotinoyle, isonicotinoyle, furoyle, thenoyle ou naphtoyle
   et X est choisi dans le groupe formé par l'hydrogène, un alcoyle en C₁-C₆, un alcoxy en C₁-C₆, un alcanoyle en C₁-C₆, un dialcoyl en C₁-C₆ amino alcoyle en C₁-C₆, un alcoxy en C₁-C₆ alcoyle en C₁-C₆, un halogène, un hydroxy, un nitro, un phényl, un phénylalcoyle en C₁-C₆, un phénoxy, un amino de la forme 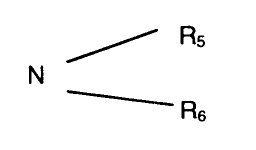 ou R₅ et R₆ sont de l'hydrogène, un radical alcoyle en C₁-C₆ éventuellement substitué ou un radical acyl en C₁-C₆, un trifluorométhyle, un trifluorométhoxy, un carboxamido ou un (alcoyle en C₁-C₆) mercapto.
- les composés de formule 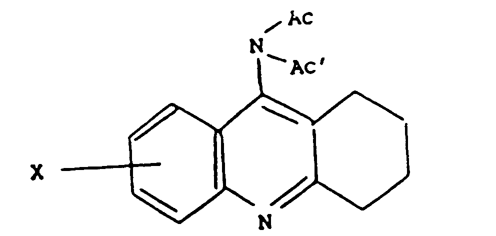 dans laquelle Ac et Ac', identiques ou différents, sont choisis dans le groupe constitué par les radicaux alcanoyle ayant de 1 à 6 atomes de carbone et les radicaux aroyle monocyclique non substitués ou substitués par 1 à 3 substituants usuels, choisis dans le groupe formé par les alcoyle inférieurs, alcoxy inférieur, halogènes, trifluorométhyle ou trifluorométhoxy et X conserve les significations antérieures.
- les composés de formule 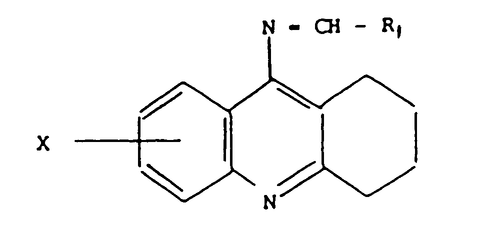 dans laquelle R₁ est un radical alcoyle inférieur, phényle ou phényl substitué par un, deux ou trois substituants choisis dans le groupe constitué par les alcoyle inférieur, les alcoxy inférieurs, les halogènes, le trifluorométhyle et le trifluorométhoxy
   et X conserve les significations antérieures
- les composés de formule 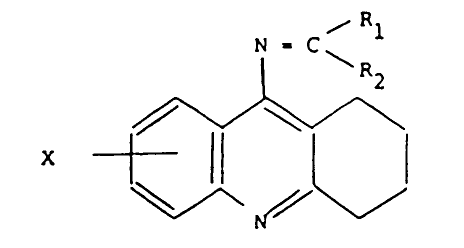 dans laquelle R₁ et R₂, identiques ou différents, sont des radicaux alcoyle inférieur, phényle ou phényle substitué par un, deux ou trois substituants ou hétérocyclique monocyclique et X conserve les signfications antérieures

La 9-amino 1,2,3,4-tétrahydroacridine et ses analogues structuraux sont préparés selon un procédé général qui consiste à soumettre un nitrile anthranilique convenablement substitué, à l'action d'une cyclohexanone en présence d'acide p.toluène sulfonique puis cyclisation en présence de diisopropylamidure de lithium selon le schéma suivant : 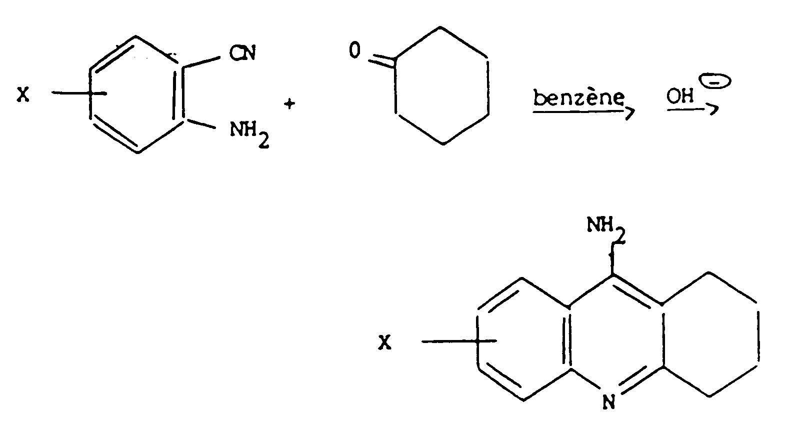
A cet égard, les analogues 8-fluoré ou 7-chloré sont parmi ceux actuellement préférés.

Au départ de la 9-amino 1,2,3,4-tétrahydroacridine, il est possible de procéder à l'alcoylation ou à l'aralcoylation de la fonction amine par action d'un aldehyde ou d'une cétone en présence d'un agent d'hydrogénation ou en présence d'acide formique.

Les bases de Schiff sont obtenues commodément par réaction de la 9-amino 1,2,3,4-tétrahydroacridine avec un aldehyde ou une cétone comme le formol, l'aldehyde benzoïque, l'aldehyde 2,6-dichloro benzoïque ou l'acétone en présence d'une base organique comme la morpholine, ou la pipéridine.

De nombreux exemples de tels dérivés sont décrits dans la demande de brevet européen 0.306.825.

La 9-amino tétrahydro acridine et ses analogues peut également être acylée à l'aide d'un dérivé fonctionnel d'acide organique carboxylique comme par exemple un halogénure d'acide, un anhydride d'acide, un anhydride mixte ou bien encore par un acide carboxylique en présence d'une carbodiimide comme la cyclohexyl carbodiimide.

Selon les concentrations d'agent acylant et la nature de solvant, il est possible de former un dérivé monoacylé ou un dérivé diacylé.

La 9-amino 1,2,3,4-tétrahydro acridine est un médicament connu déjà utilisé sous le nom de TACRINE comme antidote des agents curarisants et des produits cholinestérasiques. Son emploi a également été préconisé dans le traitement de la maladie d'Alzheimer en tant que produit anticholinestérasique et stimulant cérébral sans que son efficacité puisse être déterminée avec le recul nécessaire. Le mode d'action de ce médicament n'est pas encore établi avec certitude à en juger par le très grand nombre d'articles pharmacologiques le décrivant.

Plus récemment, J.BYRNE et T.ARIE - BMJ 298 (1989) 845 ont expliqué pourquoi la 9-amino tétrahydro acridine ne pouvait pas être utilisée en raison de son hépato nocivité qui atteint 1/5 à 1/3 des sujets traités.

Il a été trouvé maintenant que la 9-amino 1,2,3,4-Tétrahydro acridine et ses sels d'addition avec un acide minéral ou organique ainsi que ses précurseurs biologiques (pro-drugs) constituaient un médicament efficace de la régénération des lymphocytes T et en particulier des lymphocytes T4 dont le nombre décroît considérablement et dangereusement dans les cas de syndrôme immunodéficitaire.

Il apparaît donc important pour diminuer la survenue de phénomènes de toxicité, d'utiliser soit la 9-amino 1,2,3,4-tétrahydro acridine ou bien d'utiliser un de ses précurseurs dont la purification apparaît plus facile.

Il a été constaté que chez des patients dont le nombre de lymphocytes T4 avait considérablement diminué, on obtenait après quelques semaines de traitement une augmentation très importante des concentrations sanguines de lymphocytes et que chez des patients dont le nombre de lymphocytes T4 était tombé à un niveau voisin de la disparition, on obtenait dans les mêmes conditions, un retour spectaculaire à des niveaux de lymphocytes T4 subnormaux.

Cette élévation du nombre de lymphocytes T4 paraît surprenante au vue de l'art antérieur. En effet, la publication de D.J ANES et al - The Lancet (16 Avril 1988) p.887 expose le cas d'une malade de 61 ans atteinte d'une maladie d'Alzheimer et traitée pendant quatorze semaines par la tétrahydroaminoacridine associée à des doses très élevées de lécithine et à du glycopyrrolate. Chez cette malade d'un certain âge, la tétrahydroamino acridine a entraîné des phénomènes d'hépato-toxicité attestés par une hépatite granulomateuse. Chez cette malade, l'analyse des lymphocyte T a montré une diminution des sous-groupes de CD₂, CD₄ et de CD₈. On peut raisonnablement établir un parallèle entre les phénomènes d'hépato-toxicité et la diminution des populations de lymphocytes.

Au contraire, chez des sujets jeunes, la tétrahydroamino acridine n'entraîne pas de phénomènes d'hépato-nocivité et le phénomène de remontée du taux des lymphocytes n'est pas occulté ou contrecarré par une diminution anormale liée à l'arrêt de la synthèse des précurseurs sanguins des lymphocytes.

Parallèlement, les observations cliniques font état d'une régression des signes d'infection opportuniste et/ou une disparition de la séro-posivité.

La 9-amino tétrahydro acridine paraît agir principalement comme un inhibiteur de la RNA-polymérase des virus et, en particulier, des virus HIV auxquels on attribue l'origine du syndrome immuno-déficitaire.

Les précurseurs biologiques de la 9-amino tétrahydro acridine se comportent de la même façon et conduisent à la formation de 9-amino tétrahydro acridine, notamment dans l'estomac. On a constaté que dans un suc gastrique artificiel à pH 1, la méthylène 9-imino tétrahydro acridine, l'isopropylidène 9-imino tétrahydro acridine, ou la benzylidène 9-imino tétrahydro acridine étaient clivées en dérivé 9-aminé d'une façon pratiquement complète en moins de 15 mn.

La 9-amino tétrahydro acridine et ses précurseurs biologiques jouissent également de la propriété de renforcer l'action des agents antiviraux comme les dérivés de la thymidine, de l'uracile ou de l'uridine et de parmettre ainsi une diminution très importante des doses d'antiviral efficace.

C'est ainsi que l'on sait maintenant que le traitement de syndrome immuno-déficitaire par l'Azathymidine (AZT) perd progressivement son efficacité et laisse une chance de survie aux malades atteints du SIDA, seulement très limitée. Le traitement conjoint du syndrome immuno-déficitaire par un agent antiviral (AZT) et par la 9-amino tétrahydro acridine ou un de ses sels ou un de ses précurseurs biologiques a montré une possibilité de survie importante. Il est très significatif de noter à cet égard, le cas d'un malade traité pendant 15 mois par AZT avec peu de résultat chez qui le nombre des lymphocytes T4 était tombé à zéro et qui traité pendant 5 mois ensuite par la 9-amino 1,2,3,4-tétrahydro acridine était toujours en vie. Il existe de ce fait, deux possibilités de schéma thérapeutique : administration de 9-amino tétrahydro acridine seule ou administration de 9-amino tétrahydro acridine en association ou en renfort avec un agent antiviral.

Dans le cas d'agents antiviraux comme l'AZT et le HPAZI, il existe un effet synergique qui permet de réduire les doses de l'agent antiviral d'un facteur de 10 et de maintenir néanmoins l'efficacité de l'agent antiviral.

Les compositions pharmaceutiques selon l'invention renferment de 40 à 300 mg de 9-amino tétrahydro acridine ou d'un de ses sels d'addition ou d'un de ses précurseurs biologiques en association ou en mélange avec un diluant ou un excipient inerte non-toxique, pharmaceutiquement-acceptable et de préférence de 50 à 200 mg de principe actif. Comme diluant préféré, on utilise une lécithine comme celle du soja, un phospholipide comme un ganglioside ou un cérébroside ou bien une cellulose modifiée comme l'hydroxypropyl méthylcellulose.

Parmi les compositions pharmaceutiques de l'invention, renfermant à titre de principe actif soit de la 9-amino tétrahydro acridine ou un de ses sels, soit un précurseur biologique de la 9-amino tétrahydro acridine, on pourra citer celles qui sont appropriées pour l'usage par voie digestive comme les comprimés nus ou enrobés, les microgranules à libération programmée, les dragées, les gélules, les capsules molles ou dures, les tablettes, les solutions ou suspensions buvables, les gels et les émulsions.

Pour l'usage parentéral, on utilisera de préférence une solution ou une suspension de principe actif conditionnée en ampoules, en flacons multi-doses ou en seringues auto-injectables, de préférence sous forme de sels.

Pour l'usage par voie rectale, on utilisera de préférence des suppositoires ou des capsules rectales.
Les compositions pharmaceutiques selon l'invention sont préparées selon les méthodes usuelles de la pharmacotechnie.

Les exemples suivants illustrent l'invention sans toutefois la limiter :

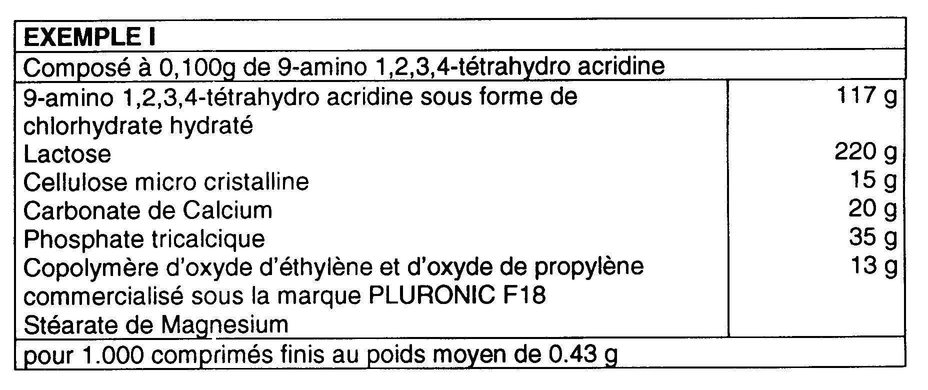

### EXEMPLE II

Expérimentation clinique de la 9-amino 1,2,3,4-tétrahydro acridine chez des malades atteints du syndrome immuno-déficitaire.

| Patient | Dose | concentration des lymphocytes T4 avant par mm³ | concentration en lymphocytes T4 après par mm³ | symptomes d'amélioration clinique |
|---|---|---|---|---|
| 1 | 100 mg/j 5 mois | 0 | 600 | Devient séro-négatif |
| 2 | 100 mg/j 3 mois | 80 | 250 | Disparition des signes d'infection |
| 3 | 100 mg/j 2 mois | 80 | 210 | Sans signe important d'infection |
| 4 | 100 mg/j 2 mois | 100 | 270 | |
| 5 | 200 mg/j 2 mois | 80 | 200 | Régression des signes d'infection opportuniste |
| La norme de T4 est de 600-800 par mm3 | | | | |

### EXEMPLE III

Détermination de l'activité inhibitrice de la 9-amino 1,2,3,4-tétrahydro acridine vis-à-vis de la RNA polymérase du virus HIV
In vitro la THA inhibe la RNA polymérase du virus HIV avec une fenêtre d'efficacité étroite.
0,1 µmole/l → 0 % inhibition
1 µmole/l → 100 % inhibition

### EXEMPLE DE PROTOCOLE CLINIQUE :

### ADULTES

| | |
|---|---|
| 1ère semaine : | 50 mg/j + 1200 mg de lécithine de soja |
| 2ème semaine : | 100 mg/j + 1200 mg de lécithine de soja |
| 3ème semaine : | 150 mg/j (si nécessaire) de lécithine de soja |
| 4ème semaine : | 200 mg/j (si nécessaire) de lécithine de soja |

### ENFANTS

Le traitement peut être débuté à raison de 10 à 25 mg/jour Le patient 1 a été traité pendant 15 mois au terme desquels, ses T4 étaients nuls.
On administre la 9-amino 1,2,3,4-tétrahydro acridine (jusqu'a 100 mg/jour). Après 5 mois de traitement, les lymphocytes T4 sont remontés et le patient était toujours en vie alors que sous AZT seul, ses chances de survie étaient minimes.

## Revendications (Revendications pour l'(les) Etat(s) contractant(s) suivant(s): AT, BE, CH, LI, DE, FR, GB, IT, LU, NL, SE)

1. Utilisation de la 9-amino 1,2,3,4-tétrahydroacridine ou d'un de ses précurseurs biologiques, seule ou associée avec un agent antiviral du type Thymidine, Uracile ou Uridine en vue de la réalisation d'un médicament assurant la regénération des lymphocytes T4 ou l'augmentation du nombre des lymphocytes T4 chez des sujets présentant un syndrome immuno-déficitaire.

2. Utilisation selon la revendication 1, dans laquelle la 9-amino 1,2,3,4-tétrahydroacridine est sous forme libre ou salifiée par un acide minéral ou organique.

3. Utilisation selon la revendication 1 ou 2, dans laquelle la teneur en principe actif s'échelonne de 10 à 300 mg par prise unitaire.

4. Utilisation selon l'une des revendications 1 à 3, dans laquelle le médicament est sous une des formes qui conviennent pour l'administration par voie parentérale, orale, percutanée, rectale ou permuqueuse.

5. Utilisation selon l'une des revendications 1à 4, dans laquelle le médicament est présenté sous forme de comprimés, de gélules ou de dragées.

6. Utilisation selon l'une des revendications 1 à 5, dans laquelle la 9-amino tétrahydroacritine est utilisée en mélange avec un diluant ou un excipient inerte, non toxique, pharmaceutiquement-acceptable.

7. Utilisation selon la revendication 6, dans laquelle le diluant est de la lécithine de soja.

8. Utilisation selon l'une des revendications 1 à 7, dans laquelle le médicament contient également de l'azidothymidine.

## Revendications (Revendications pour l'(les) Etat(s) contractant(s) suivant(s): ES, GR)

1. Un procédé d'obtention de compositions pharmaceutiques caractérisé en ce que le principe actif la 9 amino 1,2,3,4-tétrahydro acridine de formule 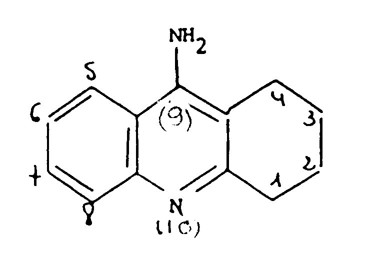 sous forme libre ou salifiée
ou un de ses précurseurs biologiques
est mis en association ou en mélange avec un véhicule ou un excipient inerte, non-toxique, pharmaceutiquement-acceptable en vue de la réalisation d'un médicament assurant la régénération des lymphocytes T4 ou l'augmentation du nombre des lymphocytes T4.

2. Procédé d'obtention selon la revendication 1, dans lequel le principe actif est la 9 amino 1,2,3,4-tétrahydroacridine sous forme libre ou salifiée par un acide minéral ou organique.

3. Un procédé d'obtention de compositions pharmaceutiques selon la revendication 1 ou la revendication 2, dans lequel la teneur en principe actif s'échelonne de 10 à 300 mg par prise unitaire.

4. Un procédé d'obtention d'une composition pharmaceutique selon l'une des revendications 1 à 3, caractérisé en ce que l'on ajoute, en outre, un agent antiviral du type Thymidine, Uracile ou Uridine.

5. Un procédé d'obtention des compositions pharmaceutiques selon l'une des revendications 1 à 4, caractérisé en ce que l'excipient inerte est un de ceux qui conviennent pour l'administration parentérale, orale, rectale, percutanée ou permuqueuse.

6. Un procédé d'obtention des compositions pharmaceutiques selon l'une des revendications 1 à 5, caractérisé en ce que le médicament contient également de l'azidothymidine

## Claims (Claims for the following Contracting State(s): AT, BE, CH, LI, DE, FR, GB, IT, LU, NL, SE)

1. Use of 9-amino 1,2,3,4-tetrahydroacridin or one of its biological precursor, alone or in association with an anti viral Thymidin ,Uracil or Uridin- like agent for the production of a drug wich ensure the regeneration of T₄ lymphocytes or the increase of the number of T₄ lymphocytes in subjects which show an immuno deficiency syndrom.

2. Use according to claim 1, in which the 9-amino 1,2,3,4-tetrahydroacridin is in the free form or salified with an organic or mineral acid.

3. Use according to claim 1 or claim 2, in which the amount of active ingredient ranges from 10 to 300 mg per unit dosage.

4. Use according to any of claims 1 to 3, in which the drug is in the form of any of those suitable for the parenteral, oral, percutaneous, rectal or permucous ways of administration.

5. Use according to any of claims 1 to 4, in which the drug is offered in the form of tablets, soft gelatin capsules or dragees.

6. Use according to any of claims 1 to 5, in which the 9-amino 1,2,3,4-tetrahydroacridin is used in mixture with a diluent or with an inert ,non toxic, pharmaceutically-acceptable carrier.

7. Use according to claim 6, in which the diluent is soya lecithin.

8. Use according to any of claims 1 to 7, in which the drug also contains azidothymidin.

## Claims (Claims for the following Contracting State(s): ES, GR)

1. A process for producing pharmaceutical compositions wherein the active ingredient of the 9-amino1,2,3,4-tetrahydroacridin having the formula 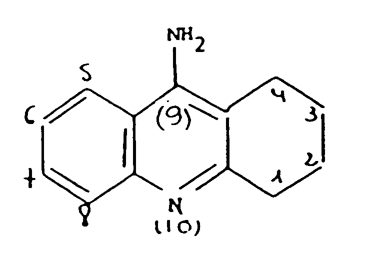 in the free or salified form
or one of its biological precursor
is in association or in a mixture with an inert, non toxic, pharmaceutically-acceptable vehicle or carrier for the production of a drug which ensure the regeneration of T₄ lymphocytes or the increase of the number of T₄ lymphocytes.

2. A process for producing according to claim 1, wherein the active ingredient is the 9-amino1,2,3,4-tetrahydroacridin in the free form or salified with an organic or mineral acid.

3. A process for producing pharmaceutical compositions according to claim 1or to claim 2,wherein the amount of active ingredient ranges from 10 to 300 mg per unit dosage.

4. A process for producing pharmaceutical compositions according to any of claims 1 to 3, wherein moreover an Thymidin, Uracil or Uridin like agent is added thereto.

5. A process for producing pharmaceutical compositions according to any of claims 1 to 4, wherein the inert carrier is one of those suitable for the parenteral, oral, rectal, percutaneous or permucous ways of administration.

6. A process for producing pharmaceutical compositions according to any of claims 1 to 5, wherein the drug also contains azidothymidin.

## Patentansprüche (Patentansprüche für folgende(n) Vertragsstaat(en): AT, BE, CH, LI, DE, FR, GB, IT, LU, NL, SE)

1. Verwendung von 9-Amino 1,2,3,4- Tetrahydroacridin oder eines von seiner biologischen Vorlaüfern, allein oder verbindet mit einem Thymidin, Uracil oder Uridin ähnliches Antiviralmittel, für die Herstellung eines Arzneimittels der eine Neubildung von den T₄ Blutkörperchen oder eine Erhöhung des Zahles von T₄ Blutkörperchen versichert bei Menschen den einen AIDS krankeit aufweisen.

2. Verwendung nach Anspruch 1 ,worin das 9-Amino 1,2,3,4- Tetrahydroacridin in der freien Form oder in der Form eines Salzes mit einer anorganischen Säure oder einer organischen Saüre vorliegt.

3. Verwendung nach Anspruch 1 oder Anspruch 2, worin der Gehalt an Wirkstoff zwischen 10 und 300 mg per Verabreichungseinheit liegt.

4. Verwendung nach einer der Ansprüche 1 bis 3, worin der Arzneimittel in einer Form ist die für die Verabreichung durch parenterale, orale, perkutaneale, rektale oder permukale Weg geeignet ist.

5. Verwendung nach einer der Ansprüche 1 bis 4, worin der Arzneimittel in der Form von Tablette, Pastille, oder Dragees vorgelegt ist.

6. Verwendung nach einer der Ansprüche 1 bis 5, worin das 9-Amino 1,2,3,4-Tetrahydroacridin in Vermischung mit einem inert, unbedenklichen, pharmazeutischverträglichen Verdünnungsmittel oder Träger, gebraucht ist.

7. Verwendung nach Ansprüch 6, worin der Verdünnungsmittel die Soya bohne Lecithin ist.

8. Verwendung nach einer der Ansprüche 1 bis 7, worin der Arzneimittel auch Azidothymidin enthält.

## Patentansprüche (Patentansprüche für folgende(n) Vertragsstaat(en): ES, GR)

1. Verfahren zur Herstellung von pharmazeutische Zusammensetzungen dadurch gekennzeichnet dass der Wirkstoff, das 9-Amino 1,2,3,4- Tetrahydroacridin der Formel 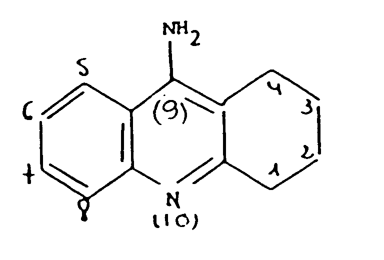 in der freien Form oder in der Form eines Salzes
oder eines von seiner biologischen Vorlaüfer
ist mit einem inerten, unbedenklichen, pharmazeutischverträglichen Vehikeln oder mit einem Träger, in Verbindung oder Vermischung gesetzt, für die Herstellung eines Arzneimittel der eine Neubildung von den T₄ Blutkörperchen oder eine Vermehrung des Zahles von T₄ Blutkörperchen versichert.

2. Verfahren zur Herstellung nach Anspruch 1, worin der Wirkstoff, das 9-Amino1,2,3,4,-Tetrahydroacridin ist, in der freien Form oder in der Form eines Salzes mit einer anorganischen oder organischen Säure.

3. Verfahren zur Herstellung von pharmazeutische Zusammensetzungen nach Anspruch1 oder Anspruch 2,worin der Gehalt an Wirkstoff von 10 bis 300 mg per Verabreichungseinheit liegt.

4. Verfahren zur Herstellung von pharmazeutische Zusammensetzungen nach einer der Ansprüche1 bis 3, worin man eine Antiviralmittel des Arts Thymidin, Uracil oder Uridin beifügt.

5. Verfahren zur Herstellung von pharmazeutische Zusammensetzungen nach einer der Ansprüche1 bis 4,worin der inerte Träger einer der die für die Verabreichung durch parenterale,
orale, rektale, perkutaneale oder permukale Weg geeignet ist.

6. Verfahren zur Herstellung von pharmazeutische Zusammensetzungen nach einer der Ansprüche1 bis 5, worin der Arzneimittel auch Azidothymidin enthält.
